# EUROPEAN PATENT APPLICATION

(11) **EP 2 316 844 A2**
(43) Date of publication of application: **04.05.2011**
(21) Application number: 10181570.2
(22) Date of filing: 18.05.2004
(51) Int. Cl.: C07K 5/00, C12N 15/00, C12N 1/21, G01N 33/00, C07K 7/08, A61K 48/00

(54) **Peptide complex**

(30) Priority: 21.05.2003 US 472131 P; 21.05.2003 EP 03011498; 23.03.2004 EP 04006900
(62) Divisional of application: 04733537.7
(71) Applicant: BIOTECH TOOLS S.A., 1120 Bruxelles (BE)
(72) Inventor: Hénot, Frederic, 1040 Bruxelles (BE); Legon, Thierry, 3360 Korbeek Lo (BE); Galy-Fauroux, Isabelle, 78670 Vilennes-sur-Seine (FR); Duchateau, Jean, 7060 Soignies (BE)
(74) Representative: von Kreisler Selting Werner

(57) **Abstract**

A complex comprising at least one heat shock protein (HSP) and at least one peptide selected from the group consisting of

| | |
|---|---|
| DYEYLINVIHAFQYV (PLP 56-70) | SEQ ID No 6 |
| EKLIETYFSKNYQDYEYLINVI (PLP 43-64) | SEQ ID No 7 |
| KTTICGKGLSATVT (PLP 104-117) | SEQ ID No 8 |
| HSLGKWLGHPDKF (PLP 139-151 C¹⁴⁰→S¹⁴⁰) | SEQ ID No 9 |
| PRHPIRVELPCRISP (MOG 8-22) | SEQ ID No 10 |
| DEGGYTCFFRDHSYQ (MOG 92-106) | SEQ ID No 11 |
| Ac-ASQKRPSQRHG (MBP ac1-11) | SEQ ID No 12 |
| TGILDSIGRFFSG (MBP 35-47) | SEQ ID No 13 |
| VHFFKNIVTPRTP (MBP 89-101) | SEQ ID No 14 |
| HCLGKWLGHPDKF (PLP 139-151) | SEQ ID No 15 |
| MEVGWYRSPFSRVVHLYRNGK (MOG) | SEQ ID No 16 |
| | |
| IYPPNANK (DER p1) | SEQ ID No 3 |
| GIEYIQHNGVVQESYYR (DER P1) | SEQ ID No 4. |

SEQUENCE LISTING

<110>  Biotech Tools S. A.

<120>  Peptide Complex

<130>  052583ep

<160>  20

<170>  PatentIn version 3.3

<210>  1
<211>  7
<212>  PRT
<213>  mammalian

<400>  1

Gly Phe Phe Tyr Thr Pro Lys
1               5


<210>  2
<211>  8
<212>  PRT
<213>  mammalian

<400>  2

Gly Phe Phe Tyr Thr Pro Lys Thr
1               5


<210>  3
<211>  8
<212>  PRT
<213>  Dermatophagoides pteronyssinus

<400>  3

Ile Tyr Pro Pro Asn Ala Asn Lys
1               5


<210>  4
<211>  17
<212>  PRT
<213>  Dermatophagoides pteronyssinus

<400>  4

Gly Ile Glu Tyr Ile Gln His Asn Gly Val Val Gln Glu Ser Tyr Tyr
1               5                   10                  15


Arg

<210>  5
<211>  8
<212>  PRT
<213>  Human immunodeficiency virus

<400>  5

Ala Ser Thr Thr Thr Asn Tyr Thr
1               5


<210>  6
<211>  15
<212>  PRT
<213>  mammalian

<400>  6

Asp Tyr Glu Tyr Leu Ile Asn Val Ile His Ala Phe Gln Tyr Val
1               5                   10                  15


<210>  7
<211>  22
<212>  PRT
<213>  mammalian

<400>  7

Glu Lys Leu Ile Glu Thr Tyr Phe Ser Lys Asn Tyr Gln Asp Tyr Glu
1               5                   10                  15


Tyr Leu Ile Asn Val Ile
            20


<210>  8
<211>  14
<212>  PRT
<213>  mammalian

<400>  8

Lys Thr Thr Ile Cys Gly Lys Gly Leu Ser Ala Thr Val Thr
1               5                   10


<210>  9
<211>  13
<212>  PRT
<213>  mammalian

<400>  9

His Ser Leu Gly Lys Trp Leu Gly His Pro Asp Lys Phe
1               5                   10


<210>  10

<211>  15
<212>  PRT
<213>  mammalian

<400>  10

Pro Arg His Pro Ile Arg Val Glu Leu Pro Cys Arg Ile Ser Pro
1               5                   10                  15


<210>  11
<211>  15
<212>  PRT
<213>  mammalian

<400>  11

Asp Glu Gly Gly Tyr Thr Cys Phe Phe Arg Asp His Ser Tyr Gln
1               5                   10                  15


<210>  12
<211>  11
<212>  PRT
<213>  mammalian

<400>  12

Ala Ser Gln Lys Arg Pro Ser Gln Arg His Gly
1               5                   10


<210>  13
<211>  13
<212>  PRT
<213>  mammalian

<400>  13

Thr Gly Ile Leu Asp Ser Ile Gly Arg Phe Phe Ser Gly
1               5                   10


<210>  14
<211>  13
<212>  PRT
<213>  mammalian

<400>  14

Val His Phe Phe Lys Asn Ile Val Thr Pro Arg Thr Pro
1               5                   10


<210>  15
<211>  13
<212>  PRT
<213>  mammalian

<400>  15

His Cys Leu Gly Lys Trp Leu Gly His Pro Asp Lys Phe
1               5                   10

<210>  16
<211>  21
<212>  PRT
<213>  mammalian

<400>  16

Met Glu Val Gly Trp Tyr Arg Ser Pro Phe Ser Arg Val Val His Leu
1               5                   10                  15

Tyr Arg Asn Gly Lys
            20

<210>  17
<211>  15
<212>  PRT
<213>  Escherichia coli

<400>  17

Gln Lys Arg Ala Ala Tyr Asp Gln Tyr Gly His Ala Ala Phe Glu
1               5                   10                  15

<210>  18
<211>  15
<212>  PRT
<213>  Homo sapiens

<400>  18

Gln Lys Arg Ala Ala Val Asp Thr Tyr Cys Arg His Asn Tyr Gly
1               5                   10                  15

<210>  19
<211>  15
<212>  PRT
<213>  mammalian

<400>  19

Gln Arg Arg Ala Ala Tyr Asp Gln Tyr Gly His Ala Ala Phe Glu
1               5                   10                  15

<210>  20
<211>  15
<212>  PRT
<213>  mammalian

<400>  20

Gln Arg Arg Ala Ala Val Asp Thr Tyr Cys Arg His Asn Tyr Gly
1               5                   10                  15

## Description

The present invention relates to complexes of heat shock proteins and tolero-genic peptides.

It is known that complexes of peptides together with heat shock proteins are suitable for inducing tolerance.

US 6,312,711 discloses a pharmaceutical or food composition intended for treating pathologies associated with graft rejection or an allergic or autoimmune reaction comprising the administration of a complex of a stress protein and epitopes of an antigenic structure.

Jack S. Burks and Kenneth P. Johnson (editors) in "Multiple sclerosis: diagnosis, medical management, and rehabilitation", Demos Medical Publishing, 2000, disclose that PLP (proteolipid protein), MOG (myelin oligodentrocytglycoprotein) and MBP (myelin basic protein) are involved in the etiology of multiple sclerosis.

Noor A. Kalsheker et al., Biochemical and Biophysical Research Communications 221, 59-61 (1996) disclose that house mite allergen DerP1 is involved in the etiology of allergen asthma.

Surprisingly, the inventors of the present invention have been able to isolate small peptides which in complex with heat shock proteins are especially useful for prevention or treatment of allergy, graft rejection or autoimmune diseases.

The inventors identified the following sequences

| | |
|---|---|
| DYEYLINVIHAFQYV (PLP 56-70) | SEQ ID No 6 |
| EKLIETYFSKNYQDYEYLINVI (PLP 43-64) | SEQ ID No 7 |
| KTTICGKGLSATVT (PLP 104-117) | SEQ ID No 8 |
| HSLGKWLGHPDKF (PLP 139-151 C¹⁴⁰→ S¹⁴⁰) | SEQ ID No 9 |
| PRHPIRVELPCRISP (MOG 8-22) | SEQ ID No 10 |
| DEGGYTCFFRDHSYQ (MOG 92-106) | SEQ ID No 11 |
| Ac-ASQKRPSQRHG (MBP ac1-11) | SEQ ID No 12 |
| TGILDSIGRFFSG (MBP 35-47) | SEQ ID No 13 |
| VHFFKNIVTPRTP (MBP 89-101) | SEQ ID No 14 |
| HCLGKWLGHPDKF (PLP 139-151) | SEQ ID No 15 |
| MEVGWYRSPFSRVVHLYRNGK (MOG) | SEQ ID No 16 |
| | |
| IYPPNANK (DER p1) | SEQ ID No 3 |
| GIEYIQHNGVVQESYYR (DER P1) | SEQ ID No 4 |
| ASTTTNYT (gp120 of HIV) | SEQ ID No 5 |
| QKRAAYDQYGHAAFE (E. Coli DnaJ) | SEQ ID No. 17 |
| | |
| QKRAAVDTYCRHNYG (HLA DRB1*0401) | SEQ ID No. 18 |
| | |
| QRRAAYDQYGHAAFE | SEQ ID No. 19 |
| and | |
| QRRAAVDTYCRHNYG | SEQ ID No. 20. |

| | |
|---|---|
| DER: Dermatophagoides pteronyssinus PLP: proteolipid protein MOG: myelin oligodendrocyte glycoprotein MBP: myelin basic protein HLA: human leukocyte antigen | |

Especially useful are sequences having the sequence

R₁ - QXRAA - R₂

with
R₁ = peptide with 1 - 10 amino acids
R₂ = peptide with 1 - 10 amino acids
X = K or R.

Preferably these sequences are prepared by hydrolysis of natural occurring peptides or proteins.

In one aspect of the invention, the invention provides a complex comprising at least one heat shock protein (HSP) and at least one peptide selected from Seq. ID Nos. 1 to 20 or having the sequence

R₁ - QXRAA - R₂

with
R₁ = peptide with 1 - 10 amino acids
R₂ = peptide with 1 - 10 amino acids
X = K or R.

In a preferred embodiment, the HSP is a bacterial HSP and more preferably a bacterial HSP from a saprophytic bacteria. Suitable HSP can be selected from hsp40, hsp70, grpE, hsp90, CPN60 (hsp60), FK506-binding proteins, gp96, calrecticulin, hsp110, grp170 and hsp100, alone or in combination.

According to the invention either complete HSP or fragments of the HSP can be used. A preferred fragment is the polypeptide binding domain of the heat shock protein.

Another aspect of the invention is a nucleic acid encoding at least one of the peptides having Seq. ID Nos. 1 to 20 or having the sequence

R₁ - QXRAA - R₂

with
R₁ = peptide with 1 - 10 amino acids
R₂ = peptide with 1 - 10 amino acids
X = K or R.

In a further aspect, the invention provides a nucleic acid encoding at least one HSP and at least one peptide comprising at least one of the sequences selected from Seq. ID No. 1 to Seq. ID No. 20 or having the sequence R₁ - QXRAA - R₂ with R₁ = peptide with 1 - 10 amino acids, R₂ = peptide with 1 - 10 amino acids, X = K or R. This nucleic acid can be used to express the corresponding peptide in vitro or in a cell or in a patient. For expression in a patient, a gene delivery vehicle would be necessary. Therefore, the gene delivery vehicle comprising at least the nucleic acid of the invention is also part of the invention.

Such a gene delivery vehicle would transfect a cell. Therefore, a cell transfected by at least one gene delivery vehicle of the invention is also part of the invention.

Either the complex or the gene delivery vehicle could be administered to patient in need thereof. Therefore, a pharmaceutical or food composition comprising the complex or the gene delivery vehicle of the present invention is also an aspect of the invention.

The complex of the present invention can be prepared by hydrolyzing *in-vitro* at least one protein with at least one protease to obtain hydrolyzed fragments and combining *in-vitro* the hydrolyzed fragments with at least one heat shock protein to obtain at least one complex.

Alternatively, the complex of the present invention can be prepared by synthesizing the peptides identified as Seq. ID No. 1 to Seq. ID No. 20 or having the sequence sequence R₁ - QXRAA - R₂ with R₁ = peptide with 1 - 10 amino acids, R₂ = peptide with 1 - 10 amino acids, X = K or R and combining the synthesized peptide with at least one heat shock protein. Suitable procedures are described by Merrifield, 1963, J. Am. Chem. Soc. 85:2149.

In a preferred embodiment, the peptide-HSP complex is formed under reducing conditions in a buffer containing at least one reducing agent. Suitable reducing agents are for example dithiothreitol and beta-mercaptoethanol. The most preferred concentrations of the reducing agent are from 0.001 mM to 700 mM, and more preferably between 0.1 mM and 50 mM.

In another embodiment, the peptide-HSP complex is formed under oxidative conditions in a buffer containing at least one oxidative agent. A suitable oxidative agent is for example hydrogen peroxide. The most preferred concentrations of the oxidative agent are from 0.1 mM to 100 mM, and more preferably between 0.5 mM and 20mM.

In another preferred embodiment, the peptide-HSP complex is formed in a buffer which contains neither a reducing agent nor an oxidative agent.

A further aspect of the invention is to use the complex of the present invention as a carrier for the delivery of biologically active molecules. The peptides can be linked to biologically active molecules e.g. nucleic acids, peptides, polypeptides, proteins, polysaccharides, lipids, drugs, and can then be combined with the heat shock protein.

In a further aspect the invention provides the isolated peptide selected from the group consisting of

| | |
|---|---|
| GFFYTPK (insulin 23-29) | SEQ ID No 1 |
| GFFYTPKT (insulin 23-30) | SEQ ID No 2 |
| IYPPNANK (DER p1) | SEQ ID No 3 |
| GIEYIQHNGVVQESYYR (DER P1) | SEQ ID No 4 |
| ASTTTNYT (gp120 of HIV) | SEQ ID No 5 |
| DYEYLINVIHAFQYV (PLP 56-70) | SEQ ID No 6 |
| EKLIETYFSKNYQDYEYLINVI (PLP 43-64) | SEQ ID No 7 |
| KTTICGKGLSATVT (PLP 104-117) | SEQ ID No 8 |
| HSLGKWLGHPDKF (PLP 139-151 C¹⁴⁰→ S¹⁴⁰) | SEQ ID No 9 |
| PRHPIRVELPCRISP (MOG 8-22) | SEQ ID No 10 |
| DEGGYTCFFRDHSYQ (MOG 92-106) | SEQ ID No 11 |
| Ac-ASQKRPSQRHG (MBP ac1-11) | SEQ ID No 12 |
| TGILDSIGRFFSG (MBP 35-47) | SEQ ID No 13 |
| VHFFKNIVTPRTP (MBP 89-101) | SEQ ID No 14 |
| HCLGKWLGHPDKF (PLP 139-151) | SEQ ID No 15 |
| MEVGWYRSPFSRVVHLYRNGK (MOG) | SEQ ID No 16 |
| QKRAAYDQYGHAAFE (E. Coli DnaJ) | SEQ ID No. 17 |
| QKRAAVDTYCRHNYG (HLA DRB1*0401) | SEQ ID No. 18 |
| QRRAAYDQYGHAAFE | SEQ ID No. 19 |
| QRRAAVDTYCRHNYG | SEQ ID No. 20 |

and

R₁ - QXRAA - R₂

with
R₁ = peptide with 1 - 10 amino acids
R₂ = peptide with 1 - 10 amino acids
X=K or R.

Also a complex of these peptides with at least one other protein such as but not limited to antibodies is part of the invention.

The complex of the present invention or the gene delivery vehicle of the invention are useful for the prevention or treatment of allergy, graft rejection or autoimmune diseases.

The complex of the present invention can also be used for the induction of a cellular reponse comprising cells able to secrete immunosuppressive cytokines after exposure to at least one antigen comprising at least one sequence selected from SEQ ID No 1 to SEQ ID No 20 or comprising the sequence R₁ - QXRAA - R₂ with R₁ = peptide with 1 - 10 amino acids, R₂ = peptide with 1 - 10 amino acids, X = K or R.

A further aspect of the invention is a diagnostic composition comprising the complex of the invention.

A further aspect of the invention is a method for the in vitro diagnosis of a disease comprising the steps of
- bringing a biological fluid of an animal containing proteins into contact with the diagnostic composition of the invention
- measuring binding of said proteins with said diagnostic composition
wherein binding indicates a disease of the animal.

Another aspect of the invention is to provide with antibodies specific for at least one of the peptides of the inventions or specific for at least one complex of the present invention. The antibody can be a polyclonal antibody, a monoclonal antibody or fragments of such as but not limited to Fab and F(ab')_{2.}

The antibody of the present invention can be used for the prevention or treatment of allergy, graft rejection or autoimmune disease. Also the antibody of the present invention can be used in a method for the in vitro diagnosis of a disease such as allergy, graft rejection or autoimmune disease.

As an alternative to direct administration of the heat shock protein peptide complex, one or more polynucleotide constructs may be administered which encode heat shock protein and target peptide in expressible form. The expressible polynucleotide constructs are introduced into cells using in vivo or in vitro methods.

Amino-acid sequences and nucleotides sequences of HSP and target proteins are generally available in sequences databases such as GenBank and Swiss-Prot.

DNA sequences can be amplified from genomic or cDNA by PCR using specific primers designed from the known sequences. "PCR" refers to the technique of polymerase chain reaction as described in Saikl, et al., Nature 324:163 (1986); and Scharf et al., Science (1986) 233:1076-1078; and US patent No. 4,683,195; and US patent No. 4,683,202. A nucleotide sequence encoding a polypeptide of any desired length can be generated by PCR. PCR can be carried out e.g. by use of a thermal cycler and a thermo-resistant DNA polymerase.

The HSP-peptide fusion protein of the invention incorporates one peptide and one heat shock protein, optionally separated by a peptide linker. Various methods for production of fusion proteins are well known in the state of the art. One suitable technique utilizes initial separate PCR amplification reactions to produce separate DNA segments encoding the two sequences, each with a linker segment attached to one end, followed by fusion of the two amplified products in a further PCR step. This technique is referred to as linker tailing. Suitable restriction sites may also be engineered into regions of interest, after which restriction digestion and ligation is used to produce the desired HSP-peptide fusion protein encoding sequence.

Methods of incorporating particular nucleic acids into expression vectors are well known to those of skill in the art, but are described in detail in, for example, Sambrook et al., Molecular Cloning: A Laboratory Manual (2nd ed.), Vols. 1 to 3, Cold Spring Harbor Laboratory, (1989) or Current Protocols in Molecular Biology, F. Asubel et al., ed. Green Publishing and Wiley-Interscience, New York (1987). To construct an expressible polynucleotide, a region encoding the heat shock protein and peptide is prepared as discussed above and inserted into a mammalian expression vector operatively linked to a suitable promoter such as the SV40 promoter, the cytomegalovirus (CMV) promoter or the Rous sarcoma virus (RSV) promoter. The resulting construct may then be used as a vaccine for genetic immunization. The nucleic acid polymer(s) could also be cloned into a viral vector. Suitable vectors include but are not limited to retroviral vectors, adenovirus vectors, vaccinia virus vectors, pox virus vectors and adenovirus-associated vectors.

Alternatively, a polynucleotide containing a sequence encoding the HSP and a gene encoding a desired peptide may be introduced respectively into two different vectors each capable of coexistence and replication in the same host.

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, complexation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

### Examples

### Protein preparation

Before enzymatic digestions, the proteins of interest are extensively washed with water by centrifugation through a centricon YM-03 or YM-10 assembly to remove any low molecular weight material loosely associated with it.

### Trypsin digestion

One to five milligrams of the protein of interest is dissolved in 1 to 5 mL of phosphate buffer 40 mM pH 8.0 (final concentration of 1 mg/mL). The solution is incubated for 10 minutes at 100°C. The solution is then rapidly cooled at 4°C, and 18 to 90 µL of ß-mercaptoethanol is added (1.8% v/v). The resulting solution is incubated at 37°C for 10 minutes and 20 to 100 µL of trypsin solution (10 mg/mL of Tris•HCl 40 mM pH 8.0; final ratio (w/w) protease/protein of 1:100) is added to the protein solution.

The resulting solution is incubated at 37°C for six hours. The solution is then centrifuged through a centricon YM-10 assembly to remove the remaining protein and trypsin.

### DnaK.ATP complex preparation

25 µL of ATP solution (4.5 mg/mL of buffer 1 (25 mM HEPES, 10 mM KCl, 3 mM magnesium chloride, 5 mM beta-mercaptoethanol, pH 7.5) is added to 400 µL of DnaK (2 mg/mL of buffer 1). The solution is incubated at 20°C for one hour, and then centrifuged through a centricon YM-10 assembly to remove any low molecular weight material loosely associated with DnaK. The large molecular weight fraction is removed, and washed extensively with buffer 1 by ultrafiltration using a centricon YM-10.

### In vitro production of stress protein-peptide complexes. Isolation of the bound peptides

The ultrafiltrated trypsin digestion is mixed with the ATP-pre-treated DnaK to give at least a 1:1 (w:w) DnaK:peptides ratio. Then, ADP is added (1 mM final) and the mixture is incubated for at least one hour at 25°C in the suitable buffer 1. The preparations are centrifuged through a centricon YM-10 assembly to remove the remaining unbound peptides. The low and the large molecular weight fractions are recovered. The large molecular weight fraction containing DnaK-peptide complexes is washed extensively with buffer 1 containing 1 mM ADP by ultrafiltration using a centricon YM-10. The bound peptides are eluted by incubating the HSP-peptide complexes in a low pH buffer. A last ultrafiltration using a centricon YM-10 is removing the large molecular weight fraction.

### HPLC analysis

The resulting low molecular weight fractions are fractionated by reverse phase high pressure liquid chromatography (HPLC) using a Vydac C18 reverse phase column (HP32, 201TP52 C18, 250/2.1 mm, 5 µm (available from Vydac)). The elution of the peptides can be monitored at both OD 214 nm and OD 280 nm.

### Biological studies

It is a model wherein NOD (Not Obese Diabetic) mice are treated after the onset of the auto-immune disease.

After the onset of the first signs of diabetes, 500 normal Langerhans islets from young NOD mice are grafted under the renal capsule of diabetic animals. Glycosuria and glycemia are then monitored daily. Mice are considered diabetic when a glucosuria is detected and glycemia exceeds 12 mmole/L (2,16 g/L) during two consecutive days. The first day of hyperglycemia is considered as the start of relapse.

### Treatment of mice

All treatments were started on the first day after the onset of the disease, that is the day before transplantation. Mice were treated by sublingual injections, one dose each two days, to achieve the total doses:
Group 1: Peptides (1 µg) + HSP (1 µg)
Group 2: Peptides (1 µg)
Group 3: HSP (1 µg)
Group 4: buffer

### Clinical outcomes

In non-treated NOD mice, the average delay before a relapse occurs is about 11 to 12 days. Considering that a delay exceeding 14 days results from a therapeutic effect, one notices that, in the group treated with complexes, the proportion of delays exceeding 14 days is 4/7 (57%). In the other group treated with either peptides, buffer or HSP alone, the proportion is 2/6 (33%). Thus, there is a therapeutic effect of the combination between an HSP and peptides given orally.

The mRNAs of interferon-γ and IL-4 have been assayed by PCR amplification in various tissues: the graft, the spleen and, as a control, the kidney. The results are expressed as ratios of the amount of interferon-γ mRNA vs the amount of actine mRNA. IL-4 was not detected in any sample, whereas interferon-γ was found in the spleen at levels that were rather similar in all the groups.

In contrast, some interesting differences were observed in the grafted islets. The level of interferon-γ was significantly lower in the group treated with peptides-HSP complexes. It is thus possible that the complexes had induced in the Peyer's patches regulatory T cells that, after their migration into the graft, have inhibited the T_{H}1 cells that contribute to the destruction of the grafted islets by secreting cytokines such as interferon-γ.

### Figures

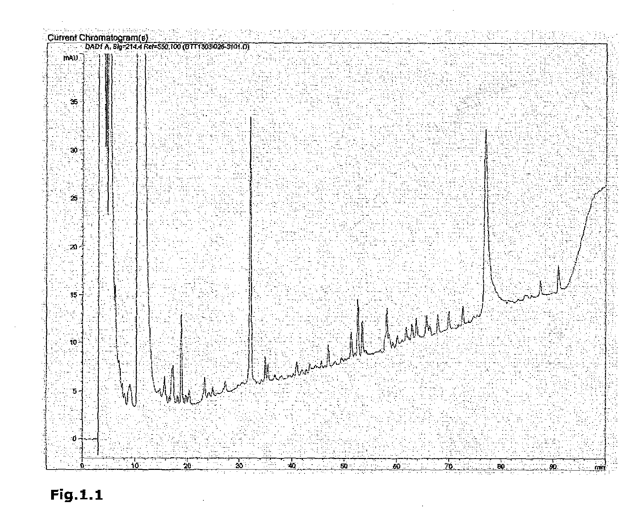

Figure 1.1: peptides (MW < or = 10 kDa) generated by trypsin-cleavage of Derp1.

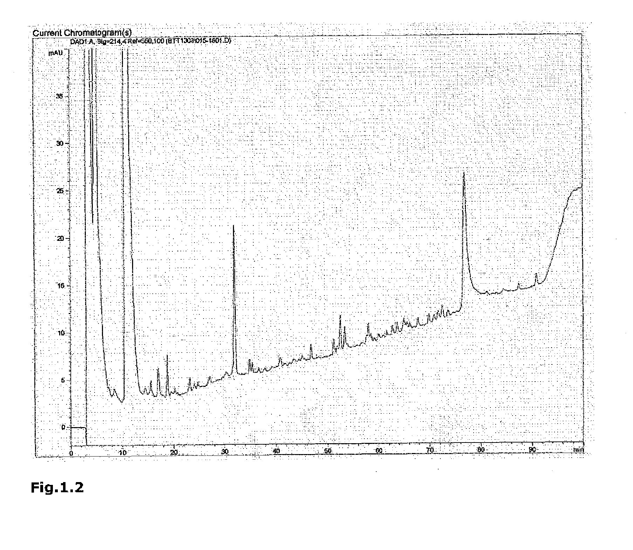

Figure 1.2: unbond peptides (MW < or 10 kDa) to DnaK, from the trypsin-cleavage of Derp1.

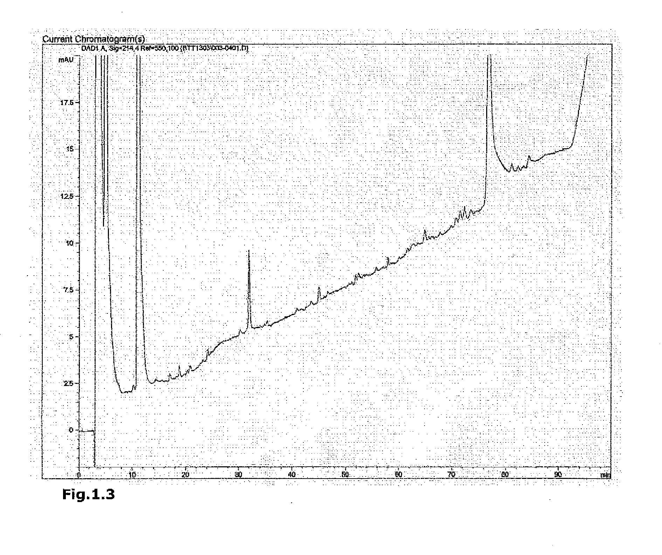

Figure 1.3: peptides from the trypsin-cleavage of Derp1 (MW < or = 10 kDa) that were bound to DnaK.

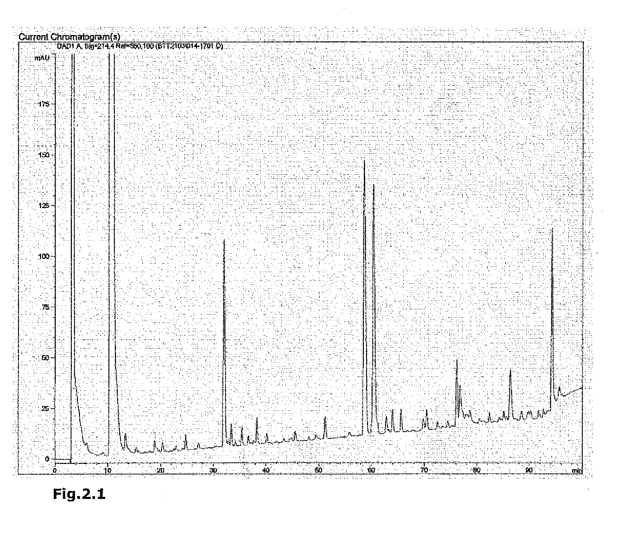

Figure 2.1: peptides (MW < or 10 kDa) generated by trypsin-cleavage of insulin.

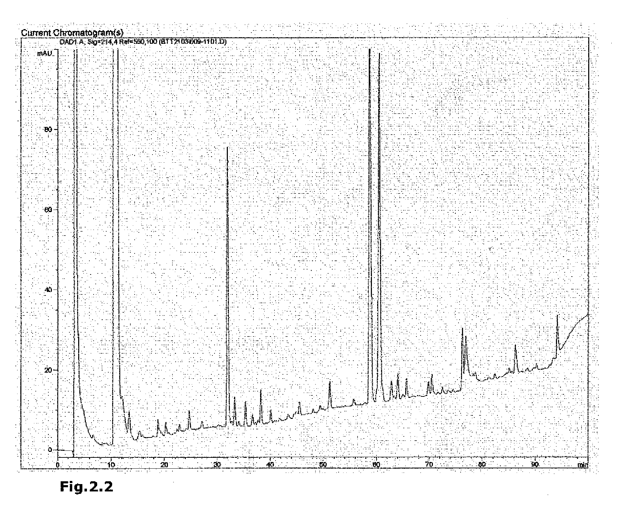

Figure 2.2: unbound peptides (MW < or 10 kDa) to DnaK, from the trypsin-cleavage of insulin.

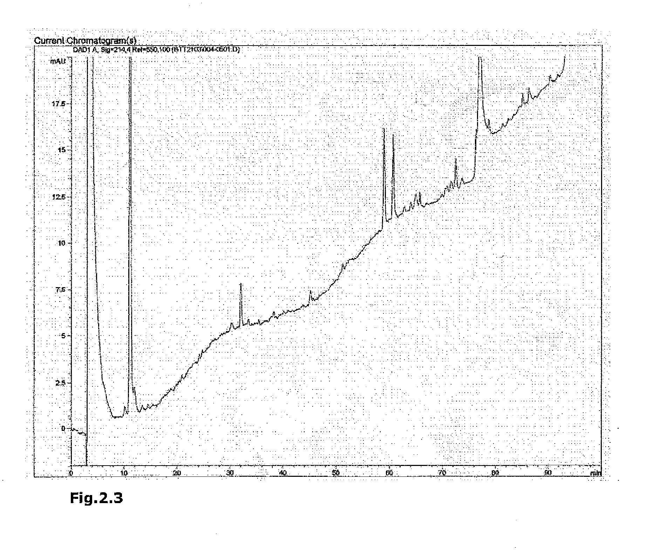

Figure 2.3: peptides from the trypsin-cleavage of insulin (MW < or = 10 kDa) that were bound to DnaK.

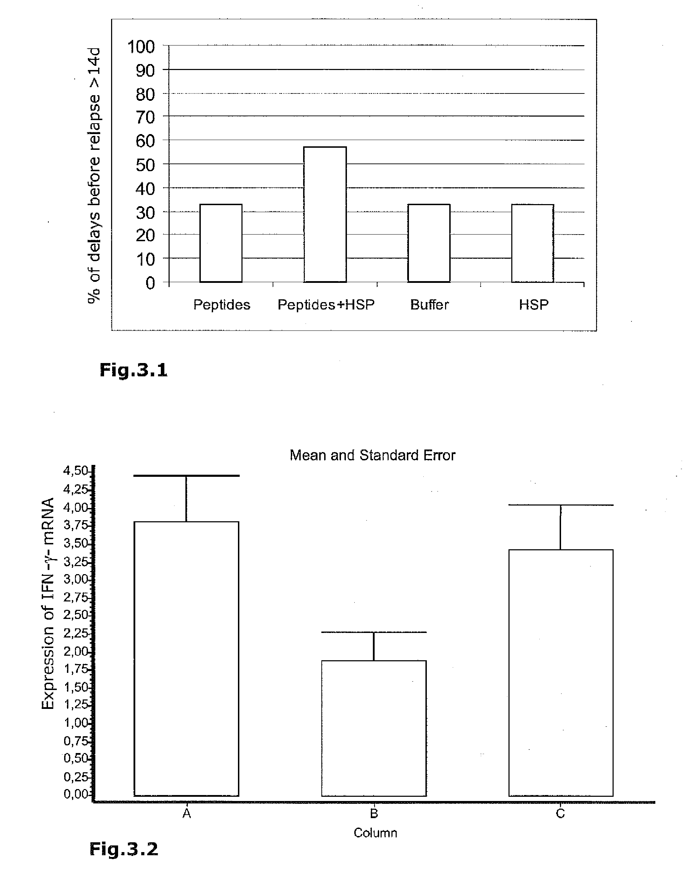

Figure 3.1: proportion of delays before relapse exceeding 14 days.
Figure 3.2: expression of IFN-γ mRNA in the grafted islets. A: group 2 (peptides); B: group 1 (HSP + peptides); C: buffer.

## Claims

1. A complex comprising at least one heat shock protein (HSP) and at least one peptide selected from the group consisting of
| | |
|---|---|
| DYEYLINVIHAFQYV (PLP 56-70) | SEQ ID No 6 |
| EKLIETYFSKNYQDYEYLINVI (PLP 43-64) | SEQ ID No 7 |
| KTTICGKGLSATVT (PLP 104-117) | SEQ ID No 8 |
| HSLGKWLGHPDKF (PLP 139-151 C¹⁴⁰→ S¹⁴⁰ | SEQ ID No 9 |
| PRHPIRVELPCRISP (MOG 8-22) | SEQ ID No 10 |
| DEGGYTCFFRDHSYQ (MOG 92-106) | SEQ ID No 11 |
| Ac-ASQKRPSQRHG (MBP ac1-11) | SEQ ID No 12 |
| TGILDSIGRFFSG (MBP 35-47) | SEQ ID No 13 |
| VHFFKNIVTPRTP (MBP 89-101) | SEQ ID No 14 |
| HCLGKWLGHPDKF (PLP 139-151) | SEQ ID No 15 |
| MEVGWYRSPFSRVVHLYRNGK (MOG) | SEQ ID No 16 |
| | |
| IYPPNANK (DER p1) | SEQ ID No 3 |
| GIEYIQHNGVVQESYYR (DER P1) | SEQ ID No 4. |

2. The complex according to claim 1, wherein the HSP is a bacterial HSP.

3. The complex according to of claim 1 or 2, wherein the HSP is selected from hsp40, hsp70, grpE, hsp90, CPN60 (hsp60), FK506-binding proteins, gp96, calrecticulin, hsp110, grp170 and hsp100.

4. The complex according to any of claims 1 to 3, wherein the heat shock protein is the polypeptide binding domain of a heat shock protein.

5. A nucleic acid encoding at least one HSP and at least one peptide comprising at least one of the sequences selected from SEQ ID No 6 to SEQ ID No 16, 3 to 4.

6. A gene delivery vehicle comprising at least one nucleic acid according to claim 5.

7. A cell transfected by at least one gene delivery vehicle according to claim 6.

8. A pharmaceutical or food compositions comprising the complex of any of claims 1 to 4 or a gene delivery vehicle of claim 6.

9. A method for preparing a complex according to any one of claims 1 to 4 comprising the steps of
• hydrolyzing *in-vitro* at least one protein with a protease to obtain hydrolyzed fragments
• combining *in-vitro* the hydrolyzed fragments with at least one heat shock protein to obtain a complex.

10. The method of claim 9, wherein unbound hydrolyzed fragments are separated from the complex.

11. Use of the complex of any of claims 1 to 4 or the gene delivery vehicle of claim 6 for the prevention or treatment of autoimmune disease.

12. Diagnostic composition comprising the complex of any of claims 1 to 4.

13. A method for the in-vitro diagnosis of a disease comprising the steps of
• bringing a biological fluid of an animal containing proteins into contact with the diagnostic composition of claim 12
• measuring binding of said proteins with said diagnostic composition
wherein binding indicates a disease of the animal.

14. An antibody binding to the complex of claims 1 to 4.

15. A host comprising two vectors, one vector containing a polynucleotide encoding HSP and one vector containing a polynucleotide encoding a desired peptide.
